# FASCICULE DE BREVET EUROPEEN

(11) **EP 0 584 199 B1**
(45) Date de publication et mention de la délivrance du brevet: **11.12.1996**
(21) Numéro de dépôt: 92911266.2
(22) Date de dépôt: 12.05.1992
(51) Int. Cl.: A61M 1/00

(54) **DISPOSITIF D'ASPIRATION POUR AUTOTRANSFUSION PER-OPERATOIRE**
ABSAUGVORRICHTUNG ZUR AUTOTRANSFUSION WAEHREND EINER OPERATION
PER-OPERATIONAL AUTOTRANSFUSION SUCTION DEVICE

(30) Priorité: 13.05.1991 FR 9105760
(43) Date de publication de la demande: 02.03.1994
(73) Titulaire: ADHOUTE, Gérard, F-83700 Saint-Raphael (FR)
(72) Inventeur: ADHOUTE, Gérard, F-83700 Saint-Raphael (FR)
(74) Mandataire: Pinguet, André
(86) Numéro de dépôt international: FR9200420
(87) Numéro de publication internationale: WO9220380

(56) Documents cités:
- EP-A- 0 400 518
- FR-A- 1 511 671
- US-A- 3 964 484
- US-A- 4 062 360

## Description

La présente invention concerne un dispositif d'aspiration pour autotransfusion per-opératoire.

La transfusion sanguine homologue, c'est-à-dire la transfusion de sang compatible provenant d'autrui, présente certains risques de transmission de maladies, telles que l'héparite ou le SIDA. Elle peut en outre induire une immunodépression et réduire les défenses naturelles du transfusé. Enfin, certains patients peuvent refuser de recevoir du sang d'autrui du fait de leurs convictions religieuses.

Pour ces raisons, la transfusion autologue ou autotransfusion, c'est-à-dire la transfusion du propre sang du transfusé, est très utilisée. Cette autotransfusion peut être différée : dans ce cas, on prélève un certain volume de sang chez le patient, et ce sang est stocké en vue d'une transfusion ultérieure au même patient. Mais le prélèvement préalable du sang n'est pas toujours possible, par exemple, lorsque le besoin de transfusion est urgent. On peut alors être amené à utiliser l'autotransfusion per-opératoire, dans laquelle le sang s'écoulant d'un patient pendant une opération chirurgicale est collecté et transfusé au même patient.

Pour cela, on utilise classiquement un dispositf comportant une canule d'aspiration reliée par un tuyau souple à un réservoir de sang mis en dépression. La canule d'aspiration est aussi reliée à un réservoir de produit anticoagulant. Pendant l'opération chirurgicale, le sang épanché au niveau du champ chirurgical est aspiré à l'aide de la canule d'aspiration, mélangé au produit anticoagulant dans ladite canule, et collecté dans le réservoir de sang avant d'être transfusé au patient.

Un problème technique primordial dans l'autotransfusion per-opératoire est d'assurer que le mélange du sang avec l'anticoagulant se fasse selon un rapport du volume d'anticoagulant au volume de sang compris entre des limites déterminées. Par exemple, avec des anticoagulants tels que le CPD (citrate-phosphate-dextrose), l'héparine ou un mélange héparine/ACD (acide citrique dextrose) [exemple de préparation de solution anticoagulante pour 1 litre de sérum physiologique : 60 ml d'AB 16 + 30 000 unités d'héparine), le rapport du volume d'anticoagulant au volume de sang recueilli doit être voisin de 1 : 7, où le mélange est optimal, et en tout cas ne doit pas être supérieur à 1 : 5 ni inférieur à 1 : 10. Si ce rapport est trop grand, la composition du sang est trop profondément modifiée pour que le sang puisse être transfusé, et s'il est trop faible, la quantité d'anticoagulant présente dans le mélange est trop faible pour empêcher la coagulation du sang. Au voisinage du rapport de 1 : 7, la composition chimique, enzymologique et morphologique globulaire du sang n'est que très légèrement modifiée, et la concentration d'anticoagulant est suffisante pour empêcher la coagulation. Avec d'autres anticoagulants, la plage de valeurs admissibles pour le rapport du volume d'anticoagulant au volume de sang pourrait être éventuellement différente.

Des données générales sur l'autotransfusion peuvent être trouvées dans les publications suivantes :
- ADHOUTE, NAHABOO, LANCELLE, MORA, ROUVIER - Autotransfusion en pratique chirurgicale. J. Chir, 1977, 114, 17-24,
- ADHOUTE, NAHABOO, LANCELLE, MORA, ROUVIER - Autotransfusion : XIIIe World Congress of the International Cardiovascular Society. 30 août, 2 septembre, Tokyo, Japon,
- ADHOUTE, NAHABOO, REYMONDON, ORSONI - Application de l'autotransfusion en chirurgie vasculaire réglée, Lyon Chir., 1978, 74, 50-62,
- ADHOUTE, NAHABOO, LANCELLE, MORA, ROUVIER, BLEYN, ORSONI - Autotransfusion in Surgical Practice, Cardio. Vasc. Res. Center Bull, 1979, 18, 40-15. Texas Medical Center, Houston, Texas 77030 - U.S.A.,
- ADHOUTE, HENIN - Economie de sang en chirurgie. J. Chirurgie, Paris, 1980, 117, 713-722,
- AUTOTRANSFUSION : Proceeding of the First International Autotransfusion Symposium : 24-25 avril, 1980. Blood Bank Laboratories, University of Maryland. School of Medecine, Maryland, U.S.A. Elsevier/North-Holland, New-York, Amsterdam, Oxford,
- ADHOUTE, AYOUB, REYMONDON, GAUTHIER - Autotransfusion peropératoire d'hémopéritoines en Chirurgie trraumatique d'urgence : J. Chir., Paris, 1988, 123, N 2, pp. 92-96, Masson, Paris, 1988,
- ADHOUTE - Autotransfusion : Utiliser son propre sang. Springer-Verlag France 26, rue des Carmes, Paris, 1989,
- ADHOUTE - Autotransfusion : Using your own blood. Springer-Verlag, Heidelberg, R.F.A., 1991.

Une solution au problème technique du contrôle de la concentration d'anticoagulant dans le sang aspiré, a été proposée dans la demande de brevet français n° 2 503 566. Ce document décrit une canule d'aspiration pour autotransfusion peropératoire ayant une première extrémité dotée d'un bec d'aspiration et une seconde extrémité reliée par un tuyau souple à un réservoir de sang maintenu en dépression par une source de vide. Une tubulure reliée à un réservoir de solution anticoagulante débouche dans la canule au voisinage du bec d'aspiration : ainsi, lorsque le bec d'aspiration aspire de l'air, la dépression dans la canule, au niveau de l'arrivée d'anticoagulant, est suffisamment faible pour ne pas aspirer d'anticoagulant; au contraire, lorsque le bec d'aspiration est plongé dans le sang au niveau du champ opératoire, l'aspiration de ce sang crée une dépression plus importante dans la canule, et donc l'anticoagulant est aspiré dans ladite canule. Les dimensions de la tubulure d'arrivée d'anticoagulant, comparées à celles du bec d'aspiration, sont telles que le rapport du volume d'anticoagulant au volume de sang aspiré sont approximativement de 1 : 7. Mais on peut noter que lorsque la canule n'est pas utilisée pour aspirer du sang, elle continue à aspirer de l'air, donc peut aussi aspirer des matériaux légers susceptibles d'obstruer le bec d'aspiration (blouse d'une personne se trouvant près du champ opératoire, par exemple) : dans ce cas, la dépression dans la canule augmente, de sorte qu'on aspire de l'anticoagulant sans aspirer de sang. Le même phénomène peut se produire pendant l'aspiration de sang, si un débris quelconque vient obstruer le bec d'aspiration, et l'utilisateur de la canule risque de ne pas s'en rendre compte immédiatement. Si le débris est entré dans le bec d'aspiration de la canule, l'utilisateur pourra être dans l'impossibilité de l'enlever rapidement : il devra alors couper la source de vide pour faire cesser l'afflux d'anticoagulant. Pendant ce temps, il est probable qu'une quantité assez importante d'anticoagulant aura été aspirée dans le réservoir de sang, de sorte que le sang contenu dans ce réservoir risque d'être inutilisable. On peut par ailleurs remarquer qu'en dehors des périodes où la canule aspire du sang, elle continue à aspirer de l'air qui passe ensuite dans le réservoir de sang avant d'être aspiré par la source de vide. On risque ainsi de concentrer dans le sang du réservoir, ou dans un filtre du réservoir, des poussières ou micro-organismes en suspension dans l'air : ceci dégrade donc l'asepsie du réservoir de sang. De plus, ce passage continu d'air au contact du sang contenu dans le réservoir est une source d'hémolyse : le sang qui sera ultérieurement transféré à l'opéré est donc appauvri en globules rouges.

Le brevet américain US-3 964 484 décrit aussi une canule d'aspiration pour autotransfusion per-opératoire, qui vise à assurer un rapport constant du volume d'anticoagulant au volume de sang aspiré. Cette canule comporte une première extrémité dotée d'un bec d'aspiration, et une seconde extrémité reliée par un tuyau souple à un réservoir de sang maintenu en dépression par une source de vide. La canule comporte en outre un orifice de mise à l'air, et est connectée à un conduit d'alimentation en anticoagulant qui débouche dans la canule au voisinage du bec d'aspiration. Le conduit d'alimentation en anticoagulant comporte une valve qui obture ledit conduit tant que la canule n'est pas mise en dépression, et ouvre ledit conduit lorsque la canule est mise en dépression. Pour cela, la valve comporte une membrane élastique souple dont un côté communique avec l'intérieur de la canule et dont l'autre côté s'applique élastiquement sur une plaque où débouche le conduit d'alimentation en anticoagulant. Tant que la canule aspire de l'air par son bec d'aspiration et/ou par son orifice de mise à l'air, l'intérieur de la canule subit une dépression faible, de sorte que la membrane souple de la valve reste apppliquée élastiquement sur sa plaque et obture le conduit d'alimentation en anticoagulant. Lorsqu'un utilisateur de la canule bouche l'orifice de mise à l'air et plonge le bec d'aspiration dans du sang, le sang est aspiré et une dépression est créée dans la canule, de sorte que la membrane souple est décollée de sa plaque et ouvre le circuit d'alimentation en anticoagulant.

Cette canule d'aspiration présente elle aussi des inconvénients :
1. En dehors des périodes où on aspire du sang, la canule aspire de l'air en permanence et cet air passe dans le réservoir de sang, avec les inconvénients cités ci-dessus.
2. Pendant l'aspiration du sang, il est possible que le bec d'aspiration soit obstrué partiellement ou totalement, par exemple par des débris divers : l'utilisateur n'a aucun moyen pour s'en apercevoir immédiatement, et donc il peut s'écouler un certain laps de temps où on aspire de l'anticoagulant et pas de sang, ou un débit de sang réduit : ceci peut conduire à rendre inutilisable le sang déjà aspiré, comme expliqué ci-dessus.

La présente invention a pour but de proposer un dispositif d'aspiration pour autotransfusion per-opératoire, qui permette de garantir que le mélange de l'anticoagulant avec le sang se fasse avec un rapport du volume d'anticoagulant au volume de sang compris dans une plage de valeurs déterminée, et qui ne dégrade pas la qualité du sang recueilli.

La présente invention a pour objet un dispositif d'aspiration pour autotransfusion per-opératoire, comportant une canule d'aspiration ayant un bec d'aspiration tubulaire communiquant avec un passage intérieur de ladite canule, ledit passage de la canule étant relié à un réservoir de collecte de sang pouvant être mis en dépression par une source de vide et ledit passage de la canule communiquant avec un circuit d'alimentation en anticoagulant comportant une valve de commande de l'arrivée d'anticoagulant, ledit dispositif étant caractérisé en ce que :
- ledit dispositif comporte un circuit de vide indépendant, destiné à aspirer uniquement de l'air et connecté à a source de vide, au réservoir de collecte de sang et à la valve,
- la valve comporte une chambre à vide reliée audit circuit de vide, ladite chambre à vide ayant une paroi déformable connectée à un obturateur du circuit d'anticoagulant, pouvant fermer le circuit d'alimentation en anticoagulant et ledit obturateur étant sollicité vers une position de fermeture par un moyen élastique et pouvant être sollicité vers une position d'ouverture par la déformation de ladite paroi déformable lorsqu'une dépression suffisante règne dans la chambre à vide, et
- le circuit de vide communique avec un orifice de mise à l'air pouvant être obturé manuellement.

L'invention a aussi pour objet une canule d'aspiration pour autotransfusion peropératoire, comportant un corps de canule formant un manche et ayant un bec d'aspiration tubulaire communiquant avec un passage intérieur de ladite canule, ledit passage de la canule pouvant être relié à un réservoir de collecte de sang qui peut être mis en dépression par une source de vide, ledit passage de la canule communiquant avec un canal d'alimentation en anticoagulant qui comporte une valve de commande de l'arrivée d'anticoagulant, caractérisée en ce que :
- ladite canule comporte un canal de vide indépendant, destiné à aspirer uniquement de l'air, connecté à la valve et pouvant être connecté à la source de vide,
- la valve comporte une chambre à vide reliée audit canal de vide, ladite chambre à vide ayant une paroi déformable connectée à un obturateur du canal d'anticoagulant, pouvant fermer le canal d'alimentation en anticoagulant, et ledit obturateur étant sollicité vers une position de fermeture par un moyen élastique et pouvant être sollicité vers une position d'ouverture par la déformation de ladite paroi déformable lorsqu'une dépression suffisante règne dans la chambre à vide, et
- le canal de vide comporte un orifice de mise à l'air pouvant être obturé manuellement.

Avantageusement, au moins une partie de la canule est transparente, de sorte qu'un utilisateur peut visualiser l'écoulement du sang dans ladite canule.

Selon une forme de réalisation avantageuse, l'obturateur du canal d'anticoagulant est déplaçable entre sa position de fermeture et une position d'ouverture maximale, en définissant ainsi une section de passage de l'anticoagulant qui varie de façon continue entre 0 et une valeur maximale.

Avantageusement, ledit moyen élastique de la valve est un ressort situé dans la chambre à vide et travaillant en compression, et la valve comporte un moyen de réglage de la force de compression du ressort.

Selon une forme de réalisation, ladite paroi déformable de la chambre à vide comporte une première face communiquant avec la chambre à vide et une deuxième face communiquant avec le circuit d'alimentation en anticoagulant. Avantageusement, l'obturateur du canal d'alimentation en anticoagulant est formé en élastomère, et la paroi déformable de la chambre à vide est une paroi souple formée en une seule pièce avec l'obturateur du canal d'alimentation en anticoagulant.

Selon une autre forme de réalisation,
- l'obturateur du canal d'anticoagulant est solidaire d'un obturateur de l'orifice de mise à l'air,
- les deux obturateurs sont déplaçables entre une position de repos, où l'obturateur du canal d'anticoagulant obture ledit canal d'anticoagulant tandis que l'obturateur de l'orifice de mise à l'air laisse dégagé l'orifice de mise à l'air, et une position d'actionnement forcé où l'obturateur du canal d'alimentation en anticoagulant ouvre au maximum le canal d'alimentation en anticoagulant tandis que l'obturateur de l'orifice de mise à l'air obture l'orifice de mise à l'air, et
- les deux obturateurs sont en liaison mécanique avec des moyens d'actionnement permettant le déplacement des deux obturateurs entre leur position de repos et leur position d'actionnement forcé.

Avantageusement, ladite paroi déformable de la chambre à vide comporte une première face communiquant avec la chambre à vide et une deuxième face soumise à la pression atmosphérique, et la canule comporte en outre une paroi déformable du circuit anticoagulant ayant une première face communiquant avec le circuit d'anticoagulant et une deuxième face soumise à la pression atmosphérique, ladite paroi déformable du circuit d'anticoagulant étant elle-aussi solidaire des deux obturateurs et ledit moyen d'actionnement est un levier qui est relié aux deux obturateurs par un premier moyen d'articulation disposé entre les deux parois déformables, ledit levier est relié au corps de la canule par un deuxième moyen d'articulation, ledit levier ayant en outre une extrémité saillant à l'extérieur du corps de la canule. Dans ce cas, selon un mode de réalisation, les deux obturateurs sont reliés par une tige ayant une section inférieure à la section des obturateurs, et ledit premier moyen d'articulation du levier est une fourche engagée sur ladite tige.

Avantageusement,
- le corps de la canule comporte un manche ayant une extrémité avant, et un ensemble de valve fixé à l'extrémité avant du manche, ledit ensemble de valve comportant un corps de base, une couronne intermédiaire et un corps de valve superposés,
- la couronne intermédiaire est traversée par un évidement cylindrique dans lequel se déplacent axialement les deux obturateurs et leur tige de liaison,
- la couronne intermédiaire comporte une fenêtre latérale qui fait communiquer l'évidement cylindrique de ladite couronne intermédiaire avec l'atmosphère, et
- le levier est moulé en une seule pièce avec ladite couronne intermédiaire, ledit levier traverse ladite fenêtre latérale et ledit deuxième moyen d'articulation est constitué par deux ponts de matière entre le levier et la couronne intermédiaire.

Selon un mode de réalisation, l'obturateur du canal d'alimentation en anticoagulant est formé en élastomère, la paroi déformable du circuit d'anticoagulant est une paroi souple formée en une seule pièce avec l'obturateur du canal d'alimentation en anticoagulant, l'obturateur de l'orifice de mise à l'air est formé en élastomère, la paroi déformable de la chambre à vide est une paroi souple formée en une seule pièce avec l'obturateur de l'orifice de mise à l'air, la paroi déformable du circuit d'anticoagulant comporte un bord extérieur périphérique qui est coincé entre le corps de base et la couronne intermédiaire, et la paroi déformable de la chambre à vide comporte un bord extérieur périphérique qui est coincé entre le corps de valve et la couronne intermédiaire.

D'autres caractéristiques et avantages de l'invention apparaîtront au cours de la description détaillée suivante d'une forme particulière de réalisation de l'invention, donnée à titre d'exemple non limitatif, en regard des dessins joints.

Sur les dessins :
- la figure 1 est une vue en coupe partielle longitudinale d'une forme de réalisation d'une canule appartenant au dispositif selon l'invention, la coupe étant prise selon la ligne I-I de la figure 3,
- la figure 2 est une vue de dessus partiellement coupée de la canule de la figure 1, la coupe étant prise selon la ligne II-II de la figure 1,
- la figure 3 est une vue d'arrière de la canule de la figure 1, vue selon la direction III de la figure 1,
- la figure 4 est une vue en perspective d'une variante de mélangeur statique pouvant être utilisé dans la canule de la figure 1,
- la figure 5 est une vue de détail en coupe et en perspective de la canule de la figure 1, la coupe étant prise selon la ligne V-V de la figure 2,
- la figure 6 est une vue schématique représentant la connexion de la canule de la figure 1 à un circuit de vide, à un réservoir de sang et à un réservoir d'anticoagulant,
- la figure 7 est une vue similaire à la figure 2, représentant une variante de la canule des figures 1 à 6,
- la figure 8 est une vue similaire à la figure 1, pour une autre forme de réalisation de la canule appartenant au dispositif selon l'invention,
- la figure 9 est une vue partielle agrandie de la canule de la figure 8, représentant le détail de la valve de la canule,
- la figure 10 est une vue en coupe de la canule des figures 8 et 9, la coupe étant prise selon la ligne X-X de la figure 9,
- la figure 10a est une vue en coupe selon la ligne A-A de la figure 10, et
- la figure 11 est une vue de détail en coupe partielle de l'obturateur de la valve de la figure 9.

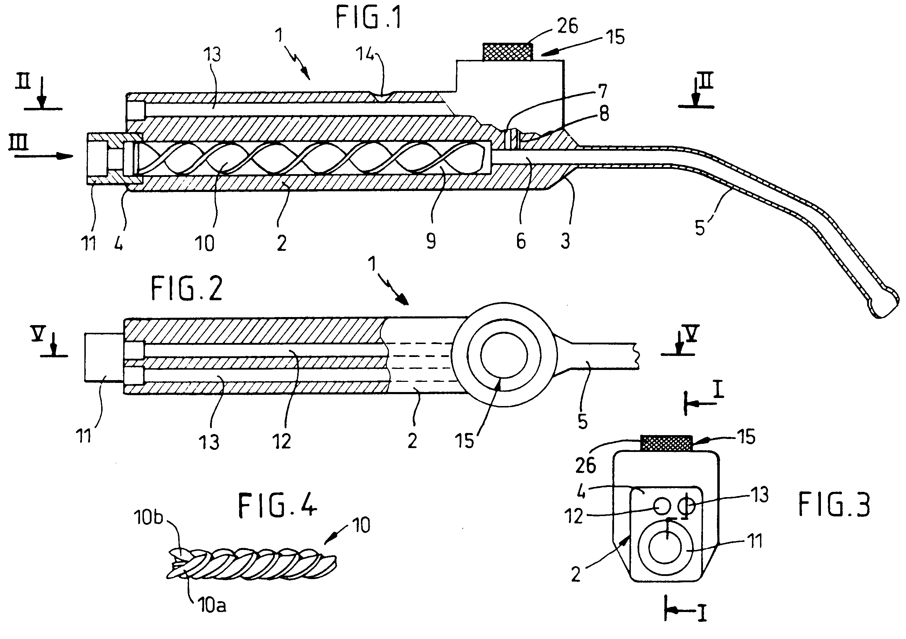

En référence à la figure 1, le dispositif d'aspiration selon l'invention comporte une canule d'aspiration 1. La canule 1 comporte un manche 2 s'étendant entre une extrémité avant 3 et une extrémité arrière 4. Avantageusement, pour des raisons qui seront vues plus loin, le manche 2 peut être réalisé en un matériau transparent, généralement en matière plastique.

L'extrémité avant 3 se prolonge par un bec d'aspiration 5 généralement en matière plastique, ayant la forme d'un conduit tubulaire recourbé vers le bas dans la position d'utilisation de la canule 1, et permettant d'aspirer du sang épanché lors d'une opération chirurgicale. Le bec d'aspiration 5 peut être formé d'une seule pièce avec le manche 2, par exemple par moulage puis coudage. Le bec d'aspiration 5 peut aussi constituer une pièce séparée qui s'emboîte dans l'extrémité avant 3 du manche 2 : l'étanchéité entre le manche 2 et le bec d'aspiration 5 peut alors se faire grâce à des joncs périphériques extérieurs du bec d'aspiration qui s'encliquètent dans des gorges complémentaires du manche 2, ou par des joints toriques disposés entre les deux pièces, ou par tout autre moyen connu; suivant le type d'opération chirurgicale réalisée, le chirurgien peut ainsi choisir un bec d'aspiration 5 ayant une forme adaptée à ses besoins. Eventuellement, la fixation du bec 5 sur le manche 2 peut être permanente (collage par exemple). Avantageusement, le bec d'aspiration 5 sera lui-même formé dans un matériau transparent.

A l'intérieur du manche 2, le bec d'aspiration communique avec un canal d'aspiration 6 longitudinal qui lui-même communique avec un canal 7 d'arrivée d'anticoagulant et un canal 8 secondaire. Le canal d'aspiration 6 se prolonge jusqu'à l'extrémité arrière 4 du manche 2 par une chambre de mélange 9. Le canal d'aspiration 6 peut être cylindrique et avoir sensiblement le même diamètre intérieur que le bec d'aspiration 5, ou bien il peut avoir une forme conique convergeant vers la chambre 9, pour des raisons qui seront vues plus loin, ou bien il peut avoir toute autre forme.

La chambre de mélange 9 peut être une cavité longitudinale cylindrique qui débouche à l'extrémité arrière 4 du manche et qui peut avoir un diamètre supérieur au diamètre du canal d'aspiration 6. La chambre de mélange 9 comporte un mélangeur statique 10, qui dans cet exemple particulier a la forme d'une hélice. En variante, comme représenté sur la figure 4, le mélangeur statique 10 pourrait être constitué par des surfaces planes réparties en deux ensembles séparés par un plan contenant l'axe de la chambre cylindrique 9 : un premier ensemble de surfaces 10a parallèles entre elles et faisant un certain angle a par rapport à l'axe de la chambre 9, et un second ensemble de surfaces 10b parallèles entre elles et non parallèles aux surfaces 10a, faisant par exemple le même angle α avec l'axe de la chambre 9. Le mélangeur statique 10 pourrait avoir toute autre forme, pourvu qu'il crée des turbulences dans l'écoulement du sang et de l'anticoagulant provenant du canal d'aspiration 6, de façon à favoriser leur mélange homogène. Eventuellement, le mélangeur 10 pourrait être non statique, par exemple constitués d'hélices tournant sous l'effet de la circulation de sang.

Des exemples de mélangeurs statiques sont donnés dans les documents US-3 964 484, US-3 955 573 et US-4 002 170.

La chambre de mélange 9 reçoit à son extrémité arrière en embout 11 qui d'une part maintient le mélangeur statique 10 dans la chambre 3, et qui d'autre part permet de connecter à la chambre 9 un tuyau souple d'évacuation du sang (non représenté) conduisant à un réservoir de collecte du sang aspiré. L'embout 11 peut être fixé au manche 2 par tout moyen, par exemple par vissage, par emboîtement à force, encliquetage, etc. Eventuellement, l'embout 11 pourrait être supprimé, la maintien du mélangeur statique 10 dans la chambre 9 se faisant alors par frottement, par collage, soudure ou autre moyen, et le tuyau souple d'évacuation du sang se connectant directement dans la chambre 9.

En référence aux figures 2 et 3, le manche 2 comporte en outre deux canaux longitudinaux qui débouchent à son extrémité arrière 4 : un canal de vide 13, qui se connecte par son extrémité arrière à un tuyau souple (non représenté) conduisant à une source de vide, et un canal d'alimentation en anticoagulant 12, qui se connecte par son extrémité arrière à un tuyau souple (non représenté) conduisant à un réservoir de produit anticoagulant. Comme représenté sur la figure 1, le canal de vide 13 communique avec un orifice de mise à l'air 14 percé en partie supérieure du manche 2 de la canule 1. En outre, les deux canaux 12 et 13 se prolongent vers une valve 15 de commande de l'arrivée d'anticoagulant, décrite ci-après de façon détaillée.

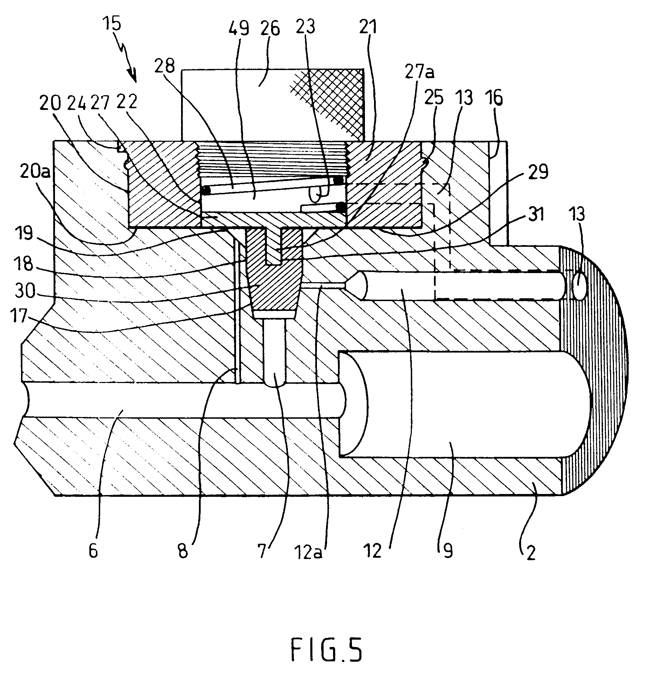

Comme représenté sur la figure 5, la valve 15 est logée dans une excroissance cylindrique 16 du manche 2, faisant saillie au-dessus du manche 2 lorsque la canule 1 est dans sa position d'utilisation, ladite excroissance 16 étant sensiblement perpendiculaire à l'axe longitudinal du manche 2 et centrée sur le canal 7 d'arrivée d'anticoagulant déjà décrit. LA canal 7 débouche dans le canal 6 d'aspiration, perpendiculairement audit canal 6. Le canal 7 s'étend sur une certaine distance vers l'excroissance 16, puis s'élargit pour former un siège de soupape 17 tronconique s'élargissant vers le haut. Le siège de soupape 17 se prolonge vers le haut par un alésage 18 cylindrique puis par un chanfrein 19 qui débouche dans un évidement 20, cylindrique ou non, en formant un épaulement 20a. L'évidement 20 débouche au sommet de l'excroissance 16. Le canal 12 d'alimentation en anticoagulant se prolonge par un tronçon 12a de section rétrécie qui débouche dans le siège de soupape 17, sensiblement perpendiculairement à l'axe dudit siège de soupape 17 tronconique. La section du tronçon 12a est prévue pour que, lorsque le siège de soupape 17 n'est pas obturé et que du sang est aspiré dans le canal 6 sous l'effet d'une dépression, on aspire par le tronçon 12a et le canal 7 un débit d'anticoagulant tel que le mélange d'anticogulant avec le sang se fasse avec un rapport du volume (ou du débit) d'anticoagulant au volume (ou au débit) de sang compris dans une plage de valeurs prédéterminée. Avec un anticogulant tel que l'héparine ou le CPD (citrate-phosphate-dextrose), on cherchera à obtenir un rapport du volume (ou du débit) d'anticoagulant au volume (ou au débit) de sang compris entre 1 : 10 et 1 : 5, et de préférence voisin de 1 : 7. Avec d'autres anticoagulants, la plage de valeurs admissibles pourra être différente. Par ailleurs, le canal de vide 13 débouche dans l'évidement 20, et le canal auxiliaire 8 relie le chanfrein 19 au canal d'aspiration 6.

Une bague 21 de forme complémentaire de l'évidement 20 est emboîtée de façon étanche dans ledit évidement 20. La bague 21 est généralement réalisée en matière plastique. La bague 21 comporte un alésage central cylindrique 22 qui est fileté en partie supérieure, et elle est percée d'un canal radial 32 qui est disposé en dessous de sa partie filetée et qui communique avec le canal 13 de vide lorsque la bague 21 est en place dans l'évidement 20. Si la bague 21 a une forme extérieure de révolution, elle comportera avantageusement un ergot extérieur 24 collaborant avec un évidement complémentaire de l'excroissance 16 pour positionner le canal radial 23 en correspondance avec le débouché du canal de vide 13 dans l'évidement 20. La fixation de la bague 21 dans l'évidement 20 peut se faire par encliquetage d'un jonc extérieur périphérique 25 de la bague 21 dans une gorge complémentaire de l'évidement 20. Eventuellement, le jonc 25 pourrait être remplacé par un joint torique périphérique s'engageant dans une gorge périphérique de l'évidement 20 et dans une gorge périphérique correspondante de la bague 21. La bague 21 peut aussi être fixée de façon permanente dans l'évidement 20, par exemple par collage. En partie supérieure, la bague 21 comporte une vis moletée 26 vissée dans la partie filetée de son alésage central 22. De plus, un disque d'appui 27 est monté coulissant dans l'alésage central 22, et comporte une tige centrale 27a s'étendant vers le siège de soupape 17. Un ressort hélicoïdal 28 est disposé entre le disque d'appui 27 et la vis moletée 26, de façon à repousser le disque d'appui 27 vers l'épaulement 20a.

Par ailleurs, une membrane élastique souple 29 est disposée entre la bague 21 et l'épaulement 20a. Cette membrane peut être simplement serrée entre la bague 21 et l'épaulement 20a, ou elle peut être collée à la bague 21. La membrane 29 pourra, par exemple, être réalisée en latex. La bague 21, la vis 26 et la membrane 29 définissent une chambre à vide 49 qui communique uniquement avec le canal de vide 13.

Enfin, la valve 15 comporte une tige de soupape 30 ayant une partie tronconique, adaptée à s'appliquer de façon étanche sur le siège de soupape 17 pour obturer le tronçon 12a du canal 12 d'alimentation en anticoagulant, et une partie cylindrique adaptée à coulisser dans l'alésage 18 cylindrique en guidant ladite tige de soupape 30. Eventuellement, l'alésage cylindrique 18 et la partie cylindrique de la tige de soupape 30 pourraient être supprimées, sans sortir du cadre de la présente invention. De plus, la tige de soupape 30 comporte un alésage central 31 dans lequel s'emboîte à force la tige 27a du disque d'appui 27, en traversant la membrane 29. La tige de soupape 30 est donc solidaire de la partie centrale de la membrane 29 et du disque d'appui 27.

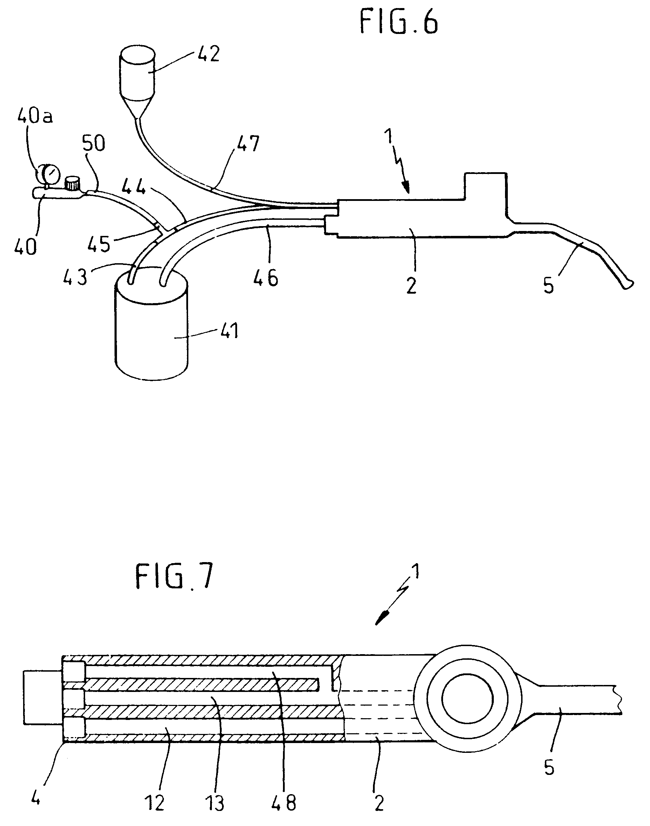

La figure 6 représente, la connexion de la canule d'aspiration 1 à la source de vide 40, au réservoir de collecte de sang 41 et au réservoir d'anticoagulant 42. La source de vide 40 qui peut être un simple robinet connecté à un circuit de vide relié à une pompe à vide ou à un autre système aspiratif, est connectée par un tuyau souple 50 à un raccord 45 en T qui la relie d'une part au réservoir de collecte de sang 41 par l'intermédiaire d'un tuyau souple 43, et d'autre part au canal de vide 13 de la canule 1 par l'intermédiaire d'un tuyau souple 44 connecté à l'extrémité arrière 4 du manche 2.

Classiquement, le réservoir de collecte de sang 41 peut être constitué d'une enveloppe extérieure rigide contenant une poche souple de collecte du sang, l'enveloppe extérieure et la poche souple étant toutes les deux reliées au tuyau souple 43. La dépression de la source de vide 40, contrôlée par un manomètre 40a pourra être de l'ordre de 30 à 60 mm de mercure, de préférence 30 à 40 mm de mercure, pour éviter le traumatisme globulaire.

Le réservoir de collecte de sang 41, et plus exactement la poche souple de collecte de sang, est connectée à la chambre de mélange 9 de la canule 1, par l'intermédiaire d'un tuyau souple 46. Le réservoir 41 comporte généralement un filtre qui retient les débris aspirés avec le sang. Le filtre pourra présenter par exemple des pores de 150 à 170 µm.En outre, le réservoir 41 est conçu, de façon connue, pour que le sang qu'il contient ne puisse pas être aspiré vers la source de vide 40.

Des exemples de réservoirs de sang utilisables dans le dispositif de la présente invention sont donnés dans les documents US-3 866 608 (ou brevet français correspondant FR-2 248 424) et US-3 680 560 (ou brevet belge correspondant BE-753 552).

Enfin, le réservoir d'anticoagulant 42 est connecté par un tuyau souple 47 au canal d'alimentation en anticoagulant 12 de la canule 1. L'anticoagulant peut être l'héparine, le CPD (citrate phosphate dextrose), un mélange héparine/ACD (acide citrique dextrose), etc.

Le fonctionnement de la canule d'aspiration est le suivant. Tant que l'orifice 14 de mise à l'air du canal de vide 13 est ouvert, de l'air est aspiré vers la source de vide 40 par l'orifice 14, le canal 13, et le tuyau 44. De ce fait, le tuyau souple 43 subit une très faible dépression, de même que le réservoir 41 de collecte du sang, et donc aucune aspiration ne se produit par l'intermédiaire du tuyau 46, de la chambre 9, du canal 6 et du bec d'aspiration 5. En outre, il ne règne aucune dépression dans la partie du canal de vide 13 comprise entre l'orifice 14 de mise à l'air et la valve 15, ou bien il y règne une dépression très faible. Ainsi, la chambre à vide 49 ne subit pas de dépression suffisante pour que la membrane soulève le disque d'appui 27 et la tige de soupape 30 : ladite tige de soupape 30 tronconique reste donc appliquée de façon étanche sur son siège de soupape 17 sous la force du ressort 28, de sorte que le tronçon 12a du canal 12 reste obturé.

Donc, tant que l'orifice 14 de mise à l'air reste libre, aucune aspiration ne se produit par le bec d'aspiration 5 de la canule 1, la canule n'est pas alimentée en anticoagulant, et en outre, il ne se produit aucune circulation d'air dans le réservoir 41 de collecte de sang. On évite ainsi l'hémolyse du sang contenu dans le réservoir 41 et sa pollution.

Si un utilisateur obture l'orifice 14 de mise à l'air avec un doigt, sans que le bec d'aspiration 5 de la canule ne plonge dans un liquide, de l'air est aspiré par le bec d'aspiration 5, le canal 6, la chambre de mélange 9, le tuyau souple 46, le réservoir 41 et de collecte de sang 41 et le tuyau 43, jusqu'à la source de vide 40. Dans ce cas précis, on fait donc circuler de l'air dans le réservoir de collecte de sang 41, mais il ne s'agit pas d'une utilisation normale de la canule d'aspiration : normalement, l'utilisateur n'obture l'orifice 14 qu'après avoir plongé le bec d'aspiration 5 dans le sang à aspirer; la circulation d'air dans le réservoir de collecte de sang n'aura donc lieu qu'exceptionnellement. D'autre part, la circulation d'air ne provoque que de faibles pertes de charge : il s'ensuit une faible dépression au niveau du raccord 45 en T, et donc une faible dépression dans le tuyau souple 44, dans le canal de vide 13 de la canule 1, et dans la chambre à vide 49 de la valve. De ce fait, la force du ressort 28 suffit à contrebalancer cette faible dépression, de sorte que la tige de soupape 30 reste appliquée contre le siège de soupape 17, et le canal 12 d'alimentation en anticoagulant reste obturé. Donc, l'aspiration d'air sans aspiration de sang ne provoque pas d'aspiration d'anticoagulant. Par conséquent, on ne risque pas de détériorer la qualité du sang déjà recueilli, par une aspiration massive d'anticoagulant.

Si maintenant l'utilisateur obture l'orifice 14 de mise à l'air après avoir plongé le bec d'aspiration 5 de la canule dans du sang épanché lors d'une opération chirurgicale, la dépression due à la source de vide 40 entraîne l'aspiration du sang jusque dans le réservoir 41 de collecte.

La circulation du sang dans la canule 1 et le tuyau 46 provoque une perte de charge importante, de sorte qu'il règne dans le tuyau 43, le raccord 45, le tuyau 44, le canal de vide 13 et la chambre à vide 49 une dépression proche de la dépression de la source de vide 40, qui est suffisante pour contrebalancer la force du ressort 28 de la valve 15. La membrane élastique souple 29 se soulève alors avec le disque d'appui 27 et la tige de soupape 30 qui se décolle ainsi de son siège de soupape 17: le canal 12 d'alimentation en anticoagulant n'est donc plus obturé, de sorte que l'anticoagulant peut s'écouler, sous l'effet de la dépression régnant dans le canal d'aspiration 6, depuis le réservoir 42 par le tuyau 47, le canal 12 et son tronçon rétréci 12a, le canal d'arrivée d'anticoagulant 7, pour aboutir dans le canal d'aspiration 6 où il commence à se mélanger avec le sang aspiré. Le mélange est homogénéisé dans la chambre de mélange 9, grâce au mélangeur statique 10. On notera que grâce à la membrane 29, l'anticoagulant ne peut pas être aspiré dans le circuit de vide.

Dans le cas où des bulles d'air sont aspirées en même temps que le sang, la dépression dans la chambre à vide 49 diminue : le ressort 28 a donc tendance à repousser la tige de soupape 30 vers son siège 17, sans pour autant l'appliquer sur le siège 17 : du fait de la forme tronconique de la tige 30 et du siège 17, ceci diminue la section de passage de l'anticoagulant entre la tige 30 et le siège 17, donc le débit d'anticoagulant arrivant dans le canal d'aspiration 6 diminue. Le débit de sang aspiré ayant lui aussi diminué du fait de la présence d'air, on arrive ainsi à maintenir un mélange de l'anticoagulant et du sang tel que le rapport du débit d'anticoagulant au débit de sang soit dans la plage de valeurs acceptable décrite précédemment. En outre, la vis moletée 26 permet à l'utilisateur de régler la force de compression du ressort 28, et donc de régler le débit d'anticoagulant.

Si le bec d'aspiration 5 vient à s'obstruer par aspiration de débris ou pour une autre raison, l'aspiration du sang cesse ou diminue : comme la canule est transparente, l'utilisateur peut s'en rendre compte facilement. En effet, la circulation du sang est visible du fait des bulles d'air ou de débris osseux ou tissulaires aspirés avec le sang, et en outre, une variation importante de la concentration en anticoagulant dans le sang aspiré ou l'aspiration d'anticoagulant seul s'accompagnerait d'un changement de couleur du liquide aspiré dans la canule. L'utilisateur peut alors réagir au plus vite et cesser d'aspirer, pour déboucher sa canule d'aspiration ou en changer.

On notera que la vis moletée 26 pourrait être omise sans sortir du cadre de la présente invention. De même, la tige de soupape 30 et son siège 17 pourraient éventuellement être simplement cylindriques au lieu d'être tronconiques, bien que cela ne soit pas préféré. Dans ce cas, le débit d'anticoagulant ne se règle pas en fonction du débit de sang comme avec la tige de soupape tronconique, et le tronçon du canal 12 est soit obturé par la tige 30, soit libéré. Par ailleurs, la membrane 29 pourrait être remplacée par un piston coulissant de façon étanche dans l'alésage 22: le disque 27 pourrait jouer ce rôle de piston, à condition de limiter sa course vers le haut pour qu'il ne s'élève pas au dessus du canal 23, auquel cas de l'anticoagulant pourrait être aspiré dans le circuit de vide. Si la membrane 29 est ainsi supprimée, le disque 27 peut être formé d'une seule pièce avec la tige de soupape 30.

On notera enfin qu'il est possible de donner au canal 6 une forme conique convergeant vers la chambre 9 : on accroît ainsi par effet Venturi la dépression régnant dans le canal 6 au droit du canal 7 d'arrivée d'anticoagulant, ce qui facilite l'aspiration de l'anticoagulant.

Lorsque l'utilisateur cesse d'obturer l'orifice 14 de mise à l'air, l'aspiration du sang cesse, et la dépression dans la chambre à vide 49 cesse aussi, de sorte que le ressort 28 repousse le disque d'appui 27, la membrane 19 et la tige de soupape 30 vers le siège de soupape 17 jusqu'à ce que ladite tige 30 soit appliquée sur ledit siège 17 en obturant le canal 12 d'alimentation en anticoagulant : l'arrivée d'anticoagulant cesse donc aussi. Dans le cas où de l'anticoagulant se serait accumulé entre la membrane 29 et l'épaulement 20a, il est collecté dans le chanfrein 19 et évacué vers le canal 6 par le canal auxiliaire 8 : il ne gêne donc pas la fermeture de la valve 15.

Sur la figure 7, on a figuré une variante de la canule selon l'invention, qui comporte un canal 48 supplémentaire, communiquant avec le canal de vide 13 et relié au réservoir 41 de collecte de sang. Dans ce cas, le tuyau 50 et le raccord 45 en T de la figure 6 sont supprimés, le tuyau 44 relie directement le canal 13 à la source de vide 40, et le tuyau 43 relie le canal 48 au réservoir de collecte de sang 41. L'orifice de mise à l'air 14 peut alors être formé soit sur le canal 13, soit sur le canal 48. Le fonctionnement du dispositif est alors le même que décrit précédemment.

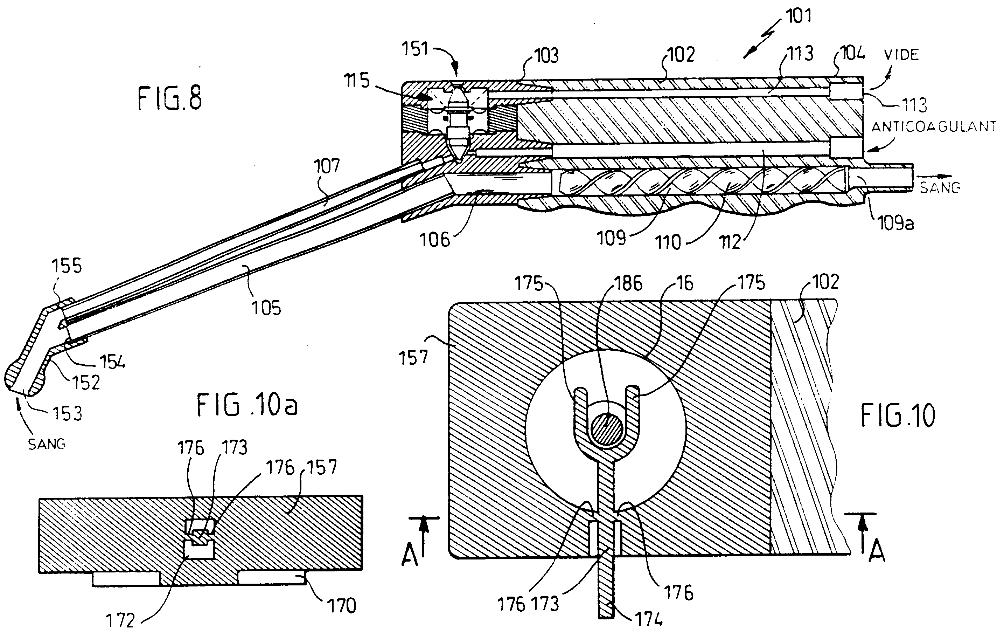

Les figures 8 à 11 représentent une autre forme de réalisation de la canule selon l'invention. La structure et le fonctionnement de cette canule sont voisins de la structure et du fonctionnement de la canule des figures 1 à 7, et donc ne seront pas décrits à nouveau en détail ici. Les éléments identiques ou similaires à ceux des figures 1 à 7 portent les mêmes références que sur les figures 1 à 7, augmentées de 100.

En référence à la figure 8, la canule 101 comporte un manche 102, réalisé par exemple en polystyrène, qui s'étend longitudinalement entre une extrémité avant 103 et une extrémité arrière 104. Le manche 102 est traversé longitudinalement par un canal de vide 113, un canal d'alimentation en anticoagulant 112, et une chambre de mélange 109. A l'extrémité arrière 104 du manche 102, le canal de vide 113 est adapté à se connecter à un tube souple (non représenté) relié à une source de vide, le canal 112 d'alimentation en anticoagulant est adapté à se connecter à un tube souple (non représenté) relié à un réservoir d'anticoagulant, et la chambre de mélange 109 est adaptée à se connecter à un tube souple (non représenté) relié à un réservoir de collecte de sang. La chambre de mélange 109 contient un mélangeur statique 110 déjà décrit, qui peut être lui-aussi réalisé en polystyrène et qui est simplement emboîté dans la chambre 109. Avantageusement, la chambre de mélange 109 comporte une extrémité arrière 109a de diamètre intérieur réduit, pour maintenir en place le mélangeur 110. En outre, la canule 101 comporte un ensemble de valve 151 qui est fixé à l'extrémité avant 103 du manche 102 et qui sera décrit plus en détail ci-dessous. L'ensemble de valve 151 comporte une extrémité avant 151a et une extrémité arrière 151b. Il contient une valve 115 de contrôle de l'alimentation en anticoagulant.

Deux tubes parallèles 105, 107, respectivement un tube ou bec d'aspiration du sang 105 et un tube d'arrivée d'anticoagulant 107, s'étendent vers l'avant à partir de l'ensemble de valve 151. Les deux tubes sont interconnectés à leur extrémité avant à l'aide d'un embout 152 d'aspiration. L'embout 152 peut comporter un orifice d'entrée 153 et deux orifices 154, 155 recevant respectivement les tubes 105 et 107. L'embout 152 et les deux tubes 105, 107 peuvent être réalisés par exemple en PVC. Les deux tubes 105, 107 peuvent être fixés dans l'embout 152 par emboîtement à force, par collage ou tout autre moyen.

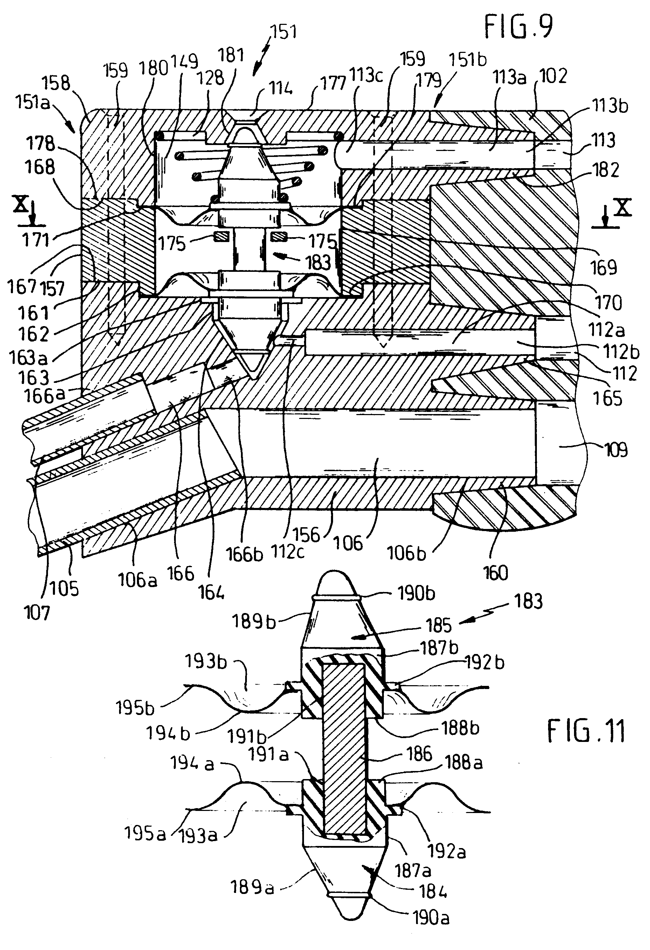

Comme représenté sur la figure 9, l'ensemble de valve 151 est constitué de trois pièces superposées : un corps de base 156 inférieur, une couronne 157 intermédiaire, et un corps de valve 158 supérieur. Ces trois pièces sont maintenues assemblées par tout moyen connu, par exemple par des vis 159 qui traversent le corps de valve 158, la couronne 157 et pénètrent dans le corps de base 156. Dans l'exemple représenté, le corps de base 156 et le corps de valve 158 sont moulés en polystyrène, tandis que la couronne intermédiaire 157 est moulée en résine acétal.

Le corps de base 156 est traversé longitudinalement par un canal d'aspiration 106, situé dans la partie inférieure du corps de base 156. Le canal d'aspiration 106 s'étend entre une extrémité avant 106a qui communique avec le tube d'aspiration du sang 105, et une extrémité arrière 106b qui communique avec la chambre de mélange 109. Le tube 105 d'aspiration du sang est fixé dans l'extrémité avant 106a du canal 106, par emboîtement à force, ou par collage. Par ailleurs, l'extrémité arrière 106b du canal 106 forme un embout 160 qui s'emboîte dans la chambre de mélange 109. L'embout 160 est fixé dans la chambre de mélange 109 par tout moyen connu, par exemple par emboîtement à force ou par collage. En outre, l'embout 160 participe au maintien du mélangeur statique 110 à l'intérieur de la chambre de mélange 109.

Le corps de base 156 comporte en outre une face supérieure 161, sur laquelle est formée une cuvette cylindrique 162. Au centre de la cuvette cylindrique 162 est formé un évidement cylindrique 163, qui se prolonge vers le bas par un évidement conique 164. Pour des raisons qui seront vues plus loin, l'évidement cylindrique 163 présente une partie supérieure élargie 163a.

En outre, le corps de base 156 comporte un canal 112a, qui s'étend entre une extrémité arrière 112b communiquant avec le canal 112 d'alimentation en anticoagulant, et une extrémité avant 112c de section réduite, qui débouche dans l'évidement conique 164. L'extrémité arrière 112b du canal 112a forme un embout 165, qui s'emboîte dans le canal 112. L'embout 165 peut être fixé dans le canal 112 par emboîtement à force, ou par collage.

Enfin, le corps de base 156 comporte un canal 166 qui s'étend entre une extrémité avant 166a dans laquelle s'emboîte le tube 107 d'arrivée d'anticoagulants, et une extrémité arrière 166b qui débouche en partie inférieure de l'évidement conique 164. Le tube 107 d'arrivée d'anticoagulants peut être fixé dans le canal 166 par tout moyen connu, par exemple par emboîtement à force ou par collage. Dans l'exemple représenté, pour des raisons qui seront vues plus loin, l'extrémité arrière 166b du canal 166 doit déboucher dans l'évidement conique 164 à une position située en dessous de la position de l'extrémité avant 112c du canal 112a.

La couronne 157 comporte une face inférieure 167 et une face supérieure 168. Elle est traversée verticalement par un évidement cylindrique 169, qui s'étend entre sa face supérieure 168 et sa face inférieure 167. La face inférieure 167 de la couronne 157 comporte une partie saillante cylindrique 170, adaptée à s'emboîter dans la cuvette 162 du corps de base. En outre, la face supérieure 168 de la couronne 157 comporte une cuvette cylindrique 171, similaire à la cuvette 162 du corps de base.

Comme représenté sur les figures 10 et 10a, la couronne 157 comporte en outre une fenêtre latérale 172, qui est traversée par un levier 173. Le levier 173 s'étend entre une extrémité extérieure 174 et une extrémité intérieure 175 formant une fourche, dans l'utilité sera vue plus loin. Le levier 173 est relié à la couronne 157 par deux ponts de matière 176 s'étendant latéralement de chaque côté du levier 173. La couronne 157 est moulée en une seule pièce avec le levier 173, avantageusement en résine acétal. Ainsi, lorsqu'un utilisateur appuie verticalement sur l'extrémité extérieure 174 du levier, comme il sera vu plus loin, les ponts de matière 176 sont déformés élastiquement par torsion, et lorsque l'utilisateur relâche sa poussée sur le levier, les ponts de matière 176 ramènent le levier 173 dans sa position initiale, par élasticité.

En référence à la figure 9, le corps de valve 158 comporte une face supérieure 177 et une face inférieure 178. La face inférieure 178 comporte un relief cylindrique 179, adapté à s'emboîter dans la cuvette cylindrique 171 de la couronne 157. En outre, le corps de valve 158 comporte un évidement cylindrique 180, formé dans le prolongement de l'évidement cylindrique 169 de la couronne 157, et qui s'étend sur certaine distance à partir de la face inférieure 178 du corps de valve. La face supérieure 177 du corps de valve comporte un orifice de mise à l'air 114, qui communique avec l'évidement cylindrique 180 du corps de valve par l'intermédiaire d'un siège de soupape 181 tronconique. En outre, le corps de valve 158 comporte un canal 113a qui s'étend entre une extrémité arrière 113b communiquant avec le canal de vide 113, et une extrémité avant 113c, qui débouche dans l'évidement cylindrique 180, ledit évidement cylindrique 180 formant ainsi une chambre à vide 149. L'extrémité arrière 113b du canal 113a forme un embout 182 qui pénètre dans le canal 113. L'embout 182 peut être fixé dans le canal 113 par tout moyen connu, par exemple par emboîtement à force ou par collage.

Un ensemble de clapet 183 est disposé à l'intérieur des évidements cylindriques 180 et 169. Comme on peut le voir sur la figure 11, l'ensemble de clapet 183 est constitué par deux pièces d'étanchéité 184, 185, reliées par une tige de liaison 186. Les deux pièces d'étanchéité 184, 185 sont moulées en élastomère, et sont avantageusement identiques. La pièce d'étanchéité 184 comporte une partie cylindrique 187a, qui s'étend axialement entre une extrémité arrière 188a et une extrémité avant conique 189a. Avantageusement, l'extrémité avant conique comporte une lèvre d'étanchéité périphérique 190a. En outre, l'extrémité arrière 188a présente un évidement cylindrique 191a. Enfin, à une position intermédiaire sur la longueur de la partie cylindrique 187a, les pièces d'étanchéité comportent une couronne extérieure 192a, qui se prolonge vers l'extérieur par un voile mince annulaire 193a. Du fait de sa minceur, le voile 193a est souple, et en outre, il est avantageusement formé avec au moins un pli annulaire 194a, pour des raisons qui seront vues plus loin. La pièce d'étanchéité 185 est identique à la pièce d'étanchéité 184 et ses différentes parties sont désignées par les mêmes références que les parties de la pièce 184, en remplaçant le sufixe "a" par "b". On notera que dans la forme de réalisation des figures 1 à 7, l'obturateur et sa membrane souple pouvaient être remplacés par une pièce d'étanchéité similaire à la pièce d'étanchéité 184.La tige 186, qui peut être moulée en polystyrène, est emboîtée dans les évidements 191 des deux pièces d'étanchéité 184, 185, et la tige 186 est fixée aux pièces d'étanchéité par tout moyen connu, par exemple par emboîtement à force, ou par collage.

Comme représenté sur la figure 9, le bord extérieur 195a de la pièce d'étanchéité 184, est coincé sur toute sa périphérie entre le fond de la cuvette 162 et la partie cylindrique 170, tandis que le bord extérieur 195b de la paroi mince de la pièce d'étanchéité supérieure 185 est coincé sur toute sa périphérie entre le fond de la cuvette 171 et la partie cylindrique 179. Ainsi, la pièce d'étanchéité supérieure 185 forme une étanchéité entre la chambre à vide 149 et l'évidement 168 de la couronne 157. De la même façon, la pièce d'étanchéité inférieure 184 forme une barrière d'étanchéité entre l'évidement 168 de la couronne 157 et le circuit d'alimentation anticoagulants.

Un ressort tronconique 128 est disposé dans la chambre à vide 149. Le ressort 128 appuie sur la couronne 192b de la pièce d'étanchéité supérieure 185, ce qui sollicite vers le bas l'ensemble de clapets 183. Ainsi, au repos, comme représenté sur la figure 9, la lèvre d'étanchéité 190 de la pièce d'étanchéité inférieure 184 est appliquée contre la surface de l'évidement conique 164, au dessus de l'extrémité 166b du canal 166, et au dessous de l'extrémité 112c du canal 112a. Donc, au repos, la pièce d'étanchéité inférieure 184 obture le canal 112 d'alimentation en anticoagulant. Dans cette position de repos, les deux branches de la fourche 175 du levier 173 sont disposées de chaque côté de la tige de liaison 186 de l'ensemble de clapets 183, et les branches de la fourche 175 sont situées à une faible distance en dessous de l'extrémité arrière 188 de la pièce d'étanchéité supérieure 185. L'élargissement supérieur 163a de l'évidement cylindrique 163, déjà décrit, a simplement pour but d'éviter une interférence entre la couronne 192a et la couronne intermédiaire 157.

Pendant le fonctionnement de la canule, lorsqu'un utilisateur obture l'orifice de mise à l'air 114 à l'aide de son doigt, il provoque à la fois une aspiration de sang et une arrivée d'anticoagulants, de la même façon qu'avec la canule des figures 1 à 7. On remarquera qu'il existe un jeu suffisant entre la fourche 175 et l'extrémité arrière 188 de la pièce d'étanchéité intérieure 184, pour permettre le libre mouvement de l'ensemble de clapets 183, sans interférence avec le levier 173. Les plis 194a et 194b des voiles 193a et 193b permettent un déplacement axial aisé de l'ensemble de clapet 183.

Lorsque l'utilisateur veut provoquer à la fois une aspiration du sang et une arrivée d'anticoagulants au débit maximum, sans utiliser les possibilités de régulation automatique de la canule selon la présente invention, il peut appuyer sur l'extrémité extérieure du levier 174. De cette façon, la fourche 175 exerce une action dirigée vers le haut sur l'ensemble de clapets 183, de sorte que la pièce d'étanchéité supérieure obture l'orifice de mise à l'air 114, par l'appui de sa lèvre d'étanchéité 190 sur le siège de soupape 181, et la pièce d'étanchéité inférieure 184 s'éloigne au maximum de sa position de repos, de sorte que l'anticoagulant peut s'écouler à débit maximum. Avantageusement, comme on peut le voir sur la figure 10, l'extrémité extérieure 174 du levier 173 est disposée sur le côté gauche de la canule, de sorte que l'utilisateur peut facilement actionner le levier 173 à l'aide de son pouce lorsqu'il tient la canule dans sa main droite.

Le dispositif selon l'invention a été testé par l'inventeur, sans retransfusion, sur un échantillon de 15 personnes, dont 5 de sexe masculin et 10 de sexe féminin. La moyenne d'age était de 57 ans (18 à 86 ans).

Les interventions chirurgicales subies par les patients sont données dans la table 1 ci-dessous.

**TABLE 1**

| | |
|---|---|
| Saphénectomie | 2 |
| Pontage fémoropoplité | 2 |
| Pontage aorto bifémoral | 2 |
| Restauration vasculaire | 4 |
| Angioplastie endoluminale | 2 |
| Splénectomie | 1 |
| Cholecystéctomie | 1 |
| Pontage | 1 |

La quantité de sang récupérée était en moyenne de 540 cm³ (de 100 à 650 cm³).

Le taux d'hémoglobine plasmatique a été mesuré dans des échantillons du sang recueilli par le dispositif selon l'invention (que nous appelerons ci-dessous sang autotransfusé) pour chaque patient. les résultats sont donnés dans la table 2 ci-dessous.

**TABLE 2**

| N° du patient | Hémoglobine plasmatique (g/dl) |
|---|---|
| 1 | 0,5 |
| 2 | 0,3 |
| 3 | 0,4 |
| 4 | 0,6 |
| 5 | 0,4 |
| 6 | 0,3 |
| 7 | 0,3 |
| 8 | 0,6 |
| 9 | 0,6 |
| 10 | 0 |
| 11 | coagulation |
| 12 | 0,1 |
| 13 | 0,3 |
| 14 | 0,2 |
| 15 | 0,4 |
| Moyenne | 0,3 |

Les produits de dégradation de là fibrine (PDF) ont été mesurés pour 14 échantillons du sang autotransfusé provenant de 14 patients (patients portant les numéros de 1 à 10 et 12 à 15).

La table 3 ci-dessous donne le nombre d'échantillons correspondant à différentes plages de valeur de PDF.

**TABLE 3**

| PDF (µg/l) | Nombre d'échantillons |
|---|---|
| < 10 | 2 |
| 10 à 20 | 1 |
| 20 à 40 | 1 |
| 40 à 80 | 2 |
| 80 à 160 | 1 |
| 160 à 320 | 2 |
| 320 à 640 | 3 |
| 640 à 2 560 | 2 |
| Total | 14 |

La table 4 ci-dessous donne la répartition de 15 échantillons de sang autotransfusé provenant respectivement des 15 patients, en fonction de la valeur de l'héparinémie (taux d'héparine dans le sang) mesuré dans ces échantillons.

**TABLE 4**

| Héparinémie U/ml | Nombre d'échantillons |
|---|---|
| < 0,05 | 4 |
| 0,09 | 1 |
| 0,28 | 1 |
| 0,40 | 1 |
| 0,41 | 1 |
| 0,7 | 1 |
| > 0,7 et < 0,8 | 3 |
| > 0,8 | 1 |
| coagulation | 1 |
| 0,1 (coagulation non controlée) | 1 |
| Total | 15 |

La table 5 ci-dessous donne la répartition de 15 échantillons de sang autotransfusé provenant respectivement des 15 patients, en fonction de la valeur du taux de prothrombine (TP) mesurée dans ces échantillons.

**TABLE 5**

| TP (%) | Nombre d'échantillons |
|---|---|
| < 10 | 9 |
| 13 | 1 |
| 26 | 1 |
| 42 | 1 |
| 44 | 1 |
| 59 | 1 |
| coagulation | 1 |
| Total | 15 |

La table 6 ci-dessous donne la comparaison de diverses mesures effectuées sur 15 échantillons de sang prélevés respectivement sur les 15 patients avec 15 échantillons de sang autotransfusé respectivement des 15 patients.

**TABLE 6**

| Mesure | Unité | Patients | | Echantillons de sang autotransfusé | |
|---|---|---|---|---|---|
| | | Moyenne | Extrêmes | Moyenne | Extrêmes |
| Hématies | x 10⁶/mm³ | 4,27 | (3,44-5,25) | 2,45 | (0,47-4,46) |
| Leucocytes | x 10³/mm³ | 9,05 | (5,4-23) | 5,67 | (1,9-12,7) |
| Hématocrite | % | 39,6 | (39,9-48) | 23,5 | (4,1-41,9) |
| Hémoglobine | g/dl | 13,31 | (11,5-15,8) | 7,72 | (1,6-13,5) |
| Plaquettes | x10³/mm³ | 294,9 | (154-513) | 89,4 | (20-196) |

Comme on peut le constater, les altérations du sang recueilli sont faibles. Le sang recueilli est relativement riche en plaquettes. Les facteurs de coagulation sont conservés et la retransfusion pourrait se faire sans difficulté.

## Revendications

1. Canule d'aspiration pour autotransfusion per-opératoire, comportant un manche (2, 102) et un bec d'aspiration (5 ; 105) avec un passage intérieur (6, 9 ; 106, 109) de ladite canule, pouvant être relié à un réservoir de collecte de sang (41) qui peut être mis en dépression, ledit passage (6, 9 ; 106, 109) de la canule communiquant avec un canal d'alimentation en anticoagulant (12, 12a ; 112, 112a) qui comporte une valve (15 ; 115) de commande de l'arrivée d'anticoagulant, caractérisée en ce que :
- ladite canule comporte un canal de vide indépendant (13 ; 113, 113a), destiné à aspirer uniquement de l'air, connecté à la valve (15 ; 115),
- la valve (15 ; 115) comporte une chambre à vide (49 ; 149) reliée audit canal de vide (13 ; 113, 113a), ladite chambre à vide ayant une paroi déformable (29 ; 193b) connectée à un obturateur (30 ; 184) du canal d'anticoagulant, pouvant fermer le canal d'alimentation en anticoagulant, et ledit obturateur étant sollicité vers une position de fermeture par un moyen élastique (28 ; 128) et pouvant être sollicité vers une position d'ouverture par la déformation de ladite paroi déformable lorsqu'une dépression suffisante règne dans la chambre à vide, et
- le canal de vide (13 ; 113, 113a) comporte un orifice de mise à l'air (14 ; 114) pouvant être obturé manuellement.

2. Canule selon la revendication 1, dans laquelle au moins une partie de la canule est transparente, de sorte qu'un utilisateur peut visualiser l'écoulement du sang dans ladite canule.

3. Canule selon la revendication 1 ou la revendication 2, dans laquelle l'obturateur du canal d'anticoagulant (30 ; 184) est déplaçable entre sa position de fermeture et une position d'ouverture maximale, en définissant ainsi une section de passage de l'anticoagulant qui varie de façon continue entre 0 et une valeur maximale.

4. Canule selon l'une quelconque des revendications 1 à 3, dans laquelle ledit moyen élastique (28) de la valve (15) est un ressort (28) situé dans la chambre à vide (49) et travaillant en compression, et la valve (15) comporte un moyen de réglage (26) de la force de compression du ressort (28).

5. Canule selon une des revendications 1 à 4, dans laquelle ladite paroi déformable (29) de la chambre à vide comporte une première face communiquant avec la chambre à vide et une deuxième face communiquant avec le canal d'alimentation en anticoagulant.

6. Canule selon une quelconque des revendications 1 à 4, dans laquelle :
- l'obturateur (184) du canal d'anticoagulant est solidaire d'un obturateur (185) de l'orifice de mise à l'air (114),
- les deux obturateurs sont déplaçables entre une position de repos, où l'obturateur (184) du canal d'anticoagulant obture ledit canal d'anticoagulant tandis que l'obturateur (185) de l'orifice de mise à l'air laisse dégagé l'orifice de mise à l'air, et une position d'actionnement forcé où l'obturateur (184) du canal d'alimentation en anticoagulant ouvre au maximum le canal d'alimentation en anticoagulant tandis que l'obturateur (185) de l'orifice de mise à l'air obture l'orifice de mise à l'air, et
- les deux obturateurs sont en liaison mécanique avec des moyens d'actionnement (173) permettant le déplacement des deux obturateurs entre leur position de repos et leur position d'actionnement forcé.

7. Canule selon la revendication 6, dans laquelle ladite paroi déformable (193b) de la chambre à vide comporte une première face communiquant avec la chambre à vide et une deuxième face soumise à la pression atmosphérique, et la canule comporte en outre une paroi déformable (193a) du circuit anticoagulant ayant une première face communiquant avec le circuit d'anticoagulant et une deuxième face soumise à la pression atmosphérique, ladite paroi déformable (193a) du circuit d'anticoagulant étant elle-aussi solidaire des deux obturateurs (184, 185) et ledit moyen d'actionnement est un levier (173) qui est relié aux deux obturateurs par un premier moyen d'articulation (175) disposé entre les deux parois déformables, ledit levier (173) est relié au corps de la canule par un deuxième moyen d'articulation (176), ledit levier (173) ayant en outre une extrémité (174) saillant à l'extérieur du corps de la canule.

8. Canule selon la revendication 7, dans laquelle les deux obturateurs (184, 185) sont reliés par une tige (186) ayant une section inférieure à la section des obturateurs, et ledit premier moyen d'articulation du levier (173) est une fourche (175) engagée sur ladite tige (186).

9. Canule selon la revendication 7 ou la revendication 8, dans laquelle :
- le manche (102) comporte une extrémité avant (103), et un ensemble de valve (151) fixé à l'extrémité avant du manche, ledit ensemble de valve comportant un corps de base (156), une couronne intermédiaire (157) et un corps de valve (158) superposés,
- la couronne intermédiaire (157) est traversée par un évidement cylindrique (168) dans lequel se déplacent axialement les deux obturateurs (184, 185) et leur tige de liaison (186),
- la couronne intermédiaire (157) comporte une fenêtre latérale (172) qui fait communiquer l'évidement cylindrique (168) de ladite couronne intermédiaire avec l'atmosphère,
- le levier (173) est moulé en une seule pièce avec ladite couronne intermédiaire (157), ledit levier traverse ladite fenêtre latérale (172) et ledit deuxième moyen d'articulation est constitué par deux ponts de matière (176) entre le levier et la couronne intermédiaire.

10. Canule selon la revendication 9, dans laquelle l'obturateur (184) du canal d'alimentation en anticoagulant est formé en élastomère, la paroi déformable (193a) du circuit d'anticoagulant est une paroi souple formée en une seule pièce avec l'obturateur (184) du canal d'alimentation en anticoagulant, l'obturateur (185) de l'orifice de mise à l'air est formé en élastomère, la paroi déformable de la chambre à vide (193b) est une paroi souple formée en une seule pièce avec l'obturateur (185) de l'orifice de mise à l'air, la paroi déformable du circuit d'anticoagulant (193a) comporte un bord extérieur périphérique (195a) qui est coincé entre le corps de base (156) et la couronne intermédiaire (157), et la paroi déformable de la chambre à vide (193b) comporte un bord extérieur périphérique qui est coincé entre le corps de valve (15!) et la couronne intermédiaire (157).

11. Canule selon la revendication 5, dans laquelle l'obturateur (184) du canal d'alimentation en anticoagulant est formé en élastomère, et la paroi déformable de la chambre à vide (193b) est une paroi souple formée en une seule pièce avec l'obturateur (184) du canal d'alimentation en anticoagulant.

## Claims

1. Suction canula for autotransfusion during surgery comprising a canula body (2, 102) and a suction nozzle (5, 105) communicating with an interior passage (6, 9, 106, 109) of said canula, said passage of the canula being connected to a blood collection storage vessel (41) adapted to be depressurized, said passage of the canula communicating with an anticoagulant feed circuit (12, 12a, 112, 112a) incorporating an anticoagulant feed control valve, wherein:
- said canula includes an independent vacuum circuit (13, 113, 113a) adapted to aspirate only air and connected to the valve (15, 115),
- the valve includes a vacuum chamber (49, 149) connected to said vacuum circuit, said vacuum chamber having a deformable wall (29, 193b) connected to a closure member (30, 184) of the anticoagulant circuit adapted to shut off the anticoagulant feed circuit and said closure member being biased towards a closed position by resilient means (28, 128) and adapted to be biased towards an open position by deformation of said deformable wall when there is a sufficient pressure drop in the vacuum chamber, and
- the vacuum circuit includes an air vent (14, 114) adapted to be shut off manually.

2. Canula according to claim 1 wherein at least part of the canula is transparent so that a user can see the blood flowing in said canula.

3. Canula according to claim 1 or to claim 2 wherein the closure member (30, 184) of the anticoagulant passage is movable between its closed position and a maximally open position to so define a cross-section of the anticoagulant passage which varies continuously between zero and a maximal value.

4. Canula according to any of claims 1 to 3 wherein said elastic means (28) of the valve (15) is a compression spring (28) disposed in the vacuum chamber (49) and the valve (15) includes means (26) for adjusting the compression force of the spring (28).

5. Canula according to any of claims 1 to 4 wherein said deformable wall (29) of the vacuum chamber has a first side communicating with the vacuum chamber and a second side communicating with the anticoagulant feed circuit.

6. Canula according to any of claims 1 to 4 wherein:
- the closure member (184) of the anticoagulant passage is fastened to a closure member (185) of the vent (114),
- both closure members are movable between a rest position in which the anticoagulant passage closure member (184) shuts off said anticoagulant passage and the air vent closure member (185) opens the air vent and a forced active position in which the anticoagulant feed passage closure member (184) opens said anticoagulant feed passage to the maximum and the air vent closure member (185) shuts off the air vent, and
- both closure members are mechanically coupled to actuator means (173) for moving the two closure members between their rest and forced active positions.

7. Canula according to claim 6 wherein said deformable wall (193b) of the vacuum chamber has a first side communicating with the vacuum chamber and a second side open to the atmosphere and the canula further comprises a deformable wall (193a) of the anticoagulant circuit having a first side communicating with the anticoagulant circuit and a second side open to the atmosphere, said deformable wall (193a) of the anticoagulant circuit being also fastened to the two closure members (184, 185), and said actuator means is a lever (173) which is connected to the two closure members by first articulation means (175) disposed between the two deformable walls and said lever (173) is connected to the body of the canula by second articulation means (176), said lever (173) also having one end (174) projecting from the body of the canula.

8. Canula according to claim 7 wherein the two closure members (184, 185) are connected by a rod (186) having a cross-section smaller than that of the closure members and said first articulation means of the lever (173) is a fork (175) engaged with said rod.

9. Canula according to claim 7 or to claim 8 wherein:
- the body (102) of the canula includes a front end (103) and a valve assembly (151) fixed to the front end of the body, said valve assembly including a base body (156), an intermediate ring (157) and a valve body (158) in a stacked arrangement,
- the two closure members (184, 185) and the rod (186) connecting them move axially in a cylindrical recess (168) extending through the intermediate ring (157),
- the intermediate ring (157) includes a lateral window (172) through which the cylindrical recess (168) of said intermediate ring is open to the atmosphere,
- the lever (173) is moulded in one piece with said intermediate ring (157), said lever passes through said lateral window (172) and said second articulation means comprise two bridges of material (176) between the lever and the intermediate ring.

10. Canula according to claim 9 wherein the anticoagulant feed passage closure member (184) is made from an elastomer material, the deformable wall (193a) of the anticoagulant circuit is a flexible wall in one piece with the anticoagulant feed passage closure member (184), the air vent closure member (185) is made from an elastomer material, the deformable wall (193b) of the vacuum chamber is a flexible wall in one piece with the air vent closure member (185), the deformable wall (193a) of the anticoagulant circuit has an outer peripheral edge (195a) trapped between the base body (156) and the intermediate ring (157) and the deformable wall of the vacuum chamber (193b) has an outer peripheral edge trapped between the valve body and the intermediate ring (157).

11. Canula according to claim 6 wherein the anticoagulant feed passage closure member (184) is made from an elastomer material and the deformable wall (193b) of the vacuum chamber is a flexible wall in one piece with the anticoagulant feed passage closure member (184).

## Patentansprüche

1. Saugkanüle für per-operative Autotransfusion, umfassend eine Hülse (2, 102) und einen Saugschnabel (5; 105), wobei eine Innenpassage (6, 9; 106, 109) dieser Kanüle an einen Blutsammelbehälter (41) angeschlossen werden kann, der unter einen Unterdruck gesetzt werden kann, wobei die Passage (6, 9; 106, 109) der Kanüle mit einem Zuführkanal für ein Antigerinnungsmittel (12, 12a; 112, 112a) kommuniziert, welcher ein Ventil (15, 115) für die Steuerung des Zutrittes des Antigerinnungsmittels umfaßt, dadurch **gekennzeichnet,** daß
- die Kanüle einen mit dem Ventil (15; 115) verbundenen unabhängigen Vakuumkanal (13; 113, 113a) umfaßt, der dazu ausgelegt ist, nur Luft anzusaugen;
- das Ventil (15; 115) eine an den Vakuumkanal (13; 113, 113a) angeschlossene Vakuumkammer (49; 149) umfaßt, wobei die Vakuumkammer eine mit einem Verschlußstück (30; 184) des Antigerinnungsmittelkanals verbundene verformbare Wand (29; 193b) hat, welche den Antigerinnungsmittelkanal verschließen kann, und wobei das Verschlußstück durch ein elastisches Mittel (28; 128) zu einer Verschließposition hin gedrückt wird und durch die Verformung der verformbaren Wand zu einer Öffnungsposition hin gedrückt werden kann, wenn ein genügender Unterdruck in der Vakuumkammer herrscht, und
- der Vakuumkanal (13; 113, 113a) eine Belüftungsöffnung (14; 114) hat, die manuell verschlossen werden kann.

2. Kanüle nach Anspruch 1, bei welcher wenigstens ein Teil der Kanüle transparent ist derart, daß ein Benutzer die Blutstromung in dieser Kanüle beobachten kann.

3. Kanüle nach Anspruch 1 oder Anspruch 2, bei welcher das Verschlußstück des Antigerinnungsmittelkanals (30; 184) zwischen seiner Verschließposition und einer maximalen Öffnungsposition verstellbar ist und so einen Durchgangsquerschnitt für das Antigerinnungsmittel definiert, welcher kontinuierlich zwischen 0 und einem Maximalwert variiert.

4. Kanüle nach einem der Ansprüche 1 bis 3, bei welcher das elastische Mittel (28) des Ventils (15) eine Feder (28) ist, die in der Vakuumkammer (49) angeordnet ist und als Druckfeder arbeitet; und daß das Ventil (15) eine Reguliereinrichtung (26) für die Druckkraft der Feder (28) aufweist.

5. Kanüle nach einem der Ansprüche 1 bis 4, bei welcher die verformbare Wand (29) der Vakuumkammer eine erste Seite umfaßt, die mit der Vakuumkammer in Verbindung steht, sowie eine zweite Seite, die mit dem Zuführkanal für das Antigerinnungsmittel in Verbindung steht.

6. Kanüle nach einem der Ansprüche 1 bis 4, bei welcher:
- das Verschlußstück (184) des Antigerinnungsmittelkanals mit einem Verschlußstück (185) der Belüftungsöffnung (114) fest verbunden ist,
- die beiden Verschlußstücke zwischen einer Ruheposition, wo das Verschlußstück (184) des Antigerinnungsmittelkanals diesen Antigerinnungsmittelkanal verschließt, während das Verschlußstück (185) der Belüftungsöffnung die Belüftungsöffnung freigibt, und einer zwangsbetätigten Position verstellbar sind, wo das Verschlußstück (184) des Antigerinnungsmittelkanals den Zuführkanal für das Antigerinnungsmittel maximal öffnet, während das Verschlußstück (185) der Belüftungsöffnung diese Belüftungsöffnung verschließt, und
- die beiden Verschlußstücke mit Betätigungsmitteln (173) mechanisch verbunden sind, welche die Verstellung der beiden Verschlußstücke zwischen ihrer Ruhestellung und ihrer zwangsbetätigten Stellung erlauben.

7. Kanüle nach Anspruch 6, bei welcher die verformbare Wand (193b) der Vakuumkammer eine erste Seite aufweist, die mit der Vakuumkammer in Verbindung steht, sowie eine zweite Seite, die dem Atmosphärendruck unterworfen ist, und wobei die Kanüle außerdem eine verformbare Wand (193a) des Antigerinnungsmittelkreises umfaßt, bei welcher eine erste Seite mit dem Antigerinnungsmittelkreis in Verbindung steht und eine zweite Seite dem Atmosphärendruck unterworfen ist, wobei diese verformbare Wand (193a) des Antigerinnungsmittelkreises ebenfalls mit den beiden Verschlußstücken (184, 185) fest verbunden ist, und wobei das Betätigungsmittel ein Hebel (173) ist, welcher mit den beiden Verschlußstücken über eine erste Gelenkanordnung (175) verbunden ist, die zwischen den beiden verformbaren Wänden angeordnet ist, wobei der Hebel (173) mit dem Korpus der Kanüle über eine zweite Gelenkanordnung (176) verbunden ist, und wobei der Hebel (173) außerdem ein Ende (174) aufweist, welches aus dem Korpus der Kanüle nach außen ragt.

8. Kanüle nach Anspruch 7, bei welcher die beiden Verschlußstücke (184, 185) über eine Stange (186) miteinander verbunden sind, welche eine gegenüber dem Querschnitt der Verschlußstücke geringeren Querschnitt hat, und wobei die erste Gelenkanordnung des Hebels (173) eine mit der Stange (186) im Eingriff befindliche Gabel (175) ist.

9. Kanüle nach Anspruch 7 oder Anspruch 8, bei welcher:
- die Hülse (102) ein Vorderende (103) aufweist sowie eine an dem Vorderende der Hülse befestigte Ventilanordnung (151), wobei die Ventilanordnung einen Basiskorpus (156), eine Zwischenkrone (157) und einen Ventilkorpus (158) aufweist, die übereinander angeordnet sind,
- die Zwischenkrone (157) von einer zylindrischen Ausnehmung (168) durchsetzt ist, in welcher sich die beiden Verschlußstücke (184, 185) und ihre Verbindungsstange (186) axial verstellen,
- die Zwischenkrone (157) ein Seitenfenster (172) aufweist, welches die zylindrische Ausnehmung (168) der Zwischenkrone mit der Atmosphäre in Verbindung bringt,
- der Hebel (173) in einem Stück mit der Zwischenkrone (157) geformt ist, wobei der Hebel durch das Seitenfenster (172) hindurchtritt und die zweite Gelenkanordnung von zwei Materialbrücken (176) zwischen dem Hebel und der Zwischenkrone gebildet ist.

10. Kanüle nach Anspruch 9, bei welcher das Verschlußstück (184) des Zuführkanals für das Antigerinnungsmittel aus einem Elastomer gebildet ist, die verformbare Wand (193a) des Antigerinnungsmittelkreises eine weiche Wand ist, die in einem Stück mit dem Verschlußstück (184) des Zuführkanals für das Antigerinnungsmittel gebildet ist, das Verschlußstück (185) der Belüftungsöffnung aus einem Elastomer gebildet ist, die verformbare Wand der Unterdruckkammer (193b) eine weiche Wand ist, die aus einem Stück mit dem Verschlußstück (185) der Belüftungsöffnung ausgebildet ist, die verformbare Wand des Antigerinnungsmittelkreises (193a) eine periphere Außenkante (195a) aufweist, die zwischen dem Basiskorpus (156) und der Zwischenkrone (157) eingeklemmt ist, und die verformbare Wand der Unterdruckkammer (193b) eine periphere Außenkante aufweist, die zwischen dem Ventilkörper (158) und der Zwischenkrone (157) eingeklemmt ist.

11. Kanüle nach Anspruch 5, bei welcher das Verschlußstück (184) des Zuführkanals für das Antigerinnungsmittel aus einem Elastomer ausgebildet ist, und die verformbare Wand der Unterdruckkammer (193b) eine weiche Wand ist, die in einem Stück mit dem Verschlußstück (184) des Zuführkanals für das Antigerinnungsmittel ausgebildet ist.
